## Europäisches Patentamt
## European Patent Office
## Office européen des brevets

(19)

(11) Veröffentlichungsnummer: **0 016 179**
**B1**

(12) **EUROPÄISCHE PATENTSCHRIFT**

(45) Veröffentlichungstag der Patentschrift: 01.06.83

(21) Anmeldenummer: 79900950.1

(22) Anmeldetag: 21.08.79

(86) Internationale Anmeldenummer:
PCT/DE79/00092

(87) Internationale Veröffentlichungsnummer:
WO 80/00441 20.03.80 Gazette 80/6

(51) Int. Cl.³: **C 07 C 9/04, C 07 C 1/00,
C 10 J 3/20**

(54) **Anlage zur Kohlevergasung.**

(30) Priorität: 31.08.78 DE 2837952

(43) Veröffentlichungstag der Anmeldung:
01.10.80 Patentblatt 80/20

(45) Bekanntmachung des Hinweises auf die Patenterteilung:
01.06.83 Patentblatt 83/22

(84) Benannte Vertragsstaaten:
AT CH DE FR GB LU NL SE

(56) Entgegenhaltungen:
DE - A - 2 553 506
DE - A - 2 704 465

I. Speiwak et al.: "Assessment of Very High-
Temperature in Process Applications (ORNL/TM
5242) published in November 1976, Oak Ridge
National Laboratory Oak Ridge, see page 82,
figure 24 (Cited in the application)

(73) Patentinhaber: GHT Gesellschaft für
Hochtemperaturreaktor-Technik mbH
Friedrich-Ebert-Strasse
D-5060 Bergisch-Gladbach 1 (DE)

(72) Erfinder: JÄGER, Walter
Schulweg 33
D 5250 Engelskirchen (DE)
Erfinder: VON WACLAWICZEK, Herbert
Friedrich-Offermann-Strasse 59
D-5060 Bergisch Gladbach 1 (DE)

(74) Vertreter: Mehl, Ernst, Dipl.-Ing. et al,
Postfach 22 01 76
D-8000 München 22 (DE)

Courier Press, Leamington Spa, England.

## Anlage zur Kohlevergasung

Die vorliegende Erfindung betrifft eine Anlage zur Erzeugung von Methan oder Synthesegas aus kohlenstoffhaltigen Stoffen, insbesondere unter Ausnutzung der Kernenergie. Dabei wird ein erster Teil des Kohlenstoffs mit Wasserstoff in einem hydrierenden Vergaser zu Methan umgesetzt und ein zweiter Teil des Kohlenstoffs mit Wasserdampf in einem Wasserdampfvergaser zu Synthesegas umgesetzt. Diese Anlagen liefern entweder Methan ($CH_4$) oder Synthesegas als Gemisch aus $H_2$ und CO. Diese Anlage ist insbesondere geeignet für Kohlen, die wenig reaktionsfreudig sind, wie z.B. Magerkohle.

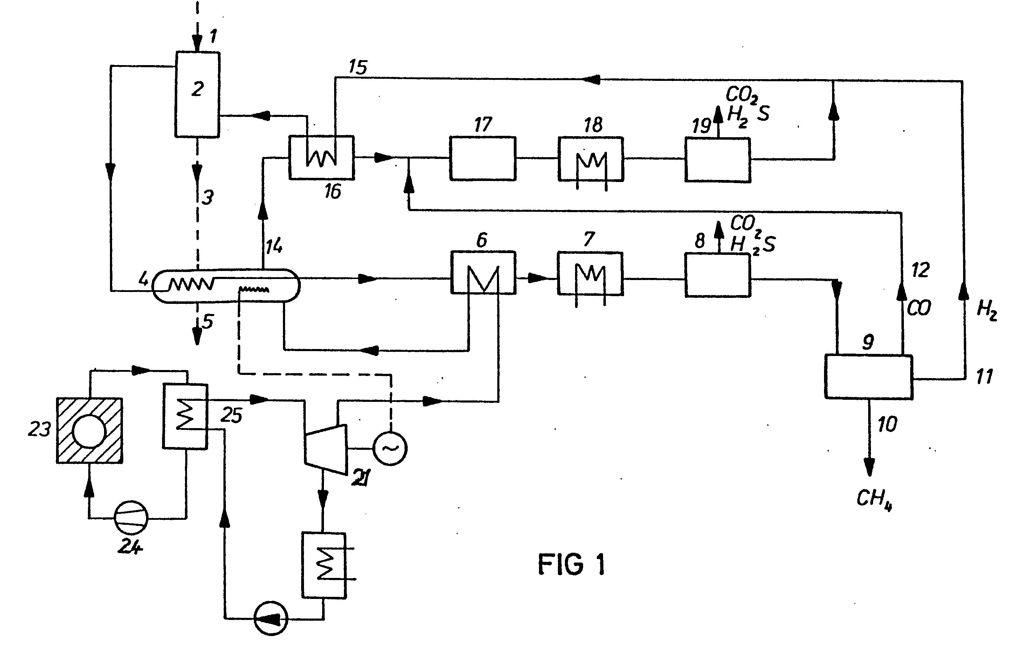

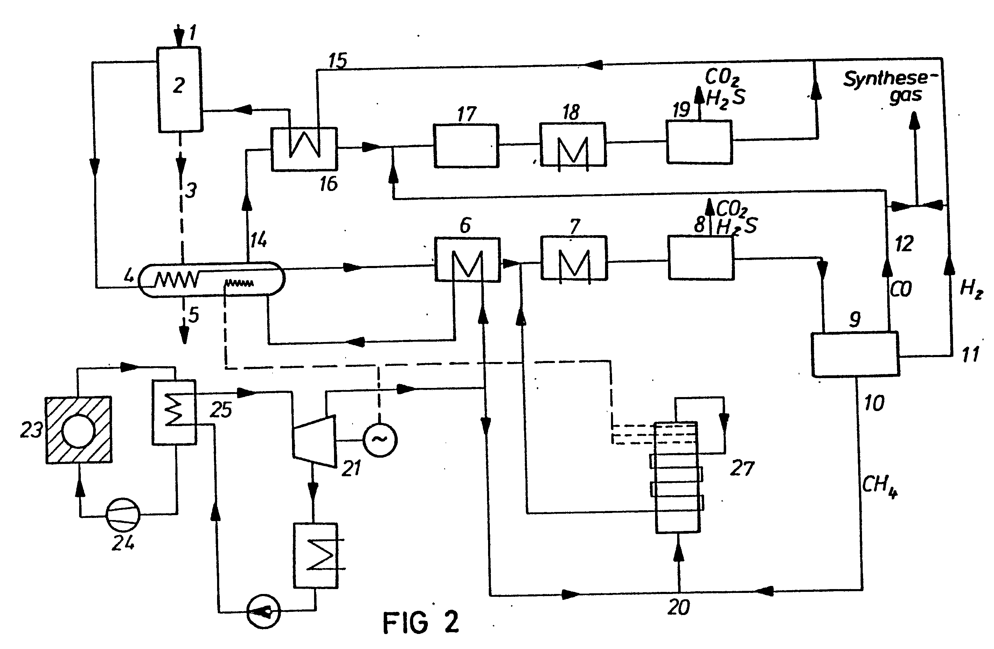

In der Zeitschrift; "Chemie-Ingenieur Technik" 1974 wird auf Seite 938, insbesondere in Abbildung 1, sowie auf Seite 941, insbesondere in Abbildung 2 je ein Prozeßschema beschrieben zur Herstellung von Methan über die Wasserdampfvergasung von Kohle. Auf Seite 937 werden neue Verfahren einer hydrierenden Vergasung von Kohle zu Methan erwähnt. Beide Verfahren sind aber mit einem erheblichen Nachteil behaftet. Bei der hydrierenden Vergasung ist wegen der großen Verweilzeiten der Kohle und mit Rücksicht auf die begrenzten Abmessungen des Vergasers keine vollkommene Umsetzung der Kohle erreichbar. Der bei der hydrierenden Vergasung abfallende Restkoks enthält außer der Asche noch ca. 30—45% des eingesetzten Kohlenstoffs. Bei der Wasserdampfvergasung dagegen kann man zwar den eingesetzten Kohlenstoff nahezu restlos vergasen. Da dieser Prozeß aber nur bei hohen Temperaturen abläuft (Steinkohle 790°C, Braunkohle 630—660°C), ist nur der obere Temperaturbereich der im Reaktor freiwerdenden Wärme für die Vergasung auszunutzen. Die restliche Wärme kann bei einem reinen Wasserdampfvergaser-Prozeß im wesentlichen nur noch zur Dampferzeugung und damit zur Stromerzeugung benutzt werden, weil nur ein kleiner Teil dieses Dampfes im Prozeß verwendet werden kann.

In dem Bericht ORNL/TM-5242 (Oak Ridge National Laboratory, November 1976) wird auf Seite 82 eine Anlage dargestellt, in der mittels nuklearer Wärme Kohlenstoff in Methan umgesetzt wird. Die Kohle wird zunächst getrocknet, dann hydrierend vergast und der Restkoks in einem Wasserdampfvergaser zu Synthesegas umgewandelt. Ein Teil des produzierten Methans wird in einem Methanspaltofen (dort als Reformer bezeichnet) unter Zusatz von heißem Wasserdampf zu Synthesegas umgesetzt. Diese Schaltung hat aber noch folgende Nachteile: Die in einem primären aber such in einem sekundären Heliumkreis angeordneten Prozeßwärmetauscher sind sehr aufwendig. Einerseits stellen Helium führende Rohrleitungen und Apparate erhöhte Anforderungen an die Dichtigkeit und andererseits muß auch verhindert werden, daß unerwünschte Stoffe auf diese Weise in den Primärkreis eindringen und dort entweder korrodierend wirken, unerwünschte Ablagerungen bilden oder derart aktiviert werden, daß sie an anderer Stelle stören, Außerdem haben alle mit reinem Helium beheizten Prozeßwärmetauscher den Nachteil, daß Helium als einatomiges Gas keine Strahlungswärme abgibt, was besonders bei den hier vorgesehenen hohen Temperaturen von Nachteil ist. Man müßte also die Heliumgeschwindigkeit erhöhen, um den Wärmeübergang durch Konvektion zu erhöhen, was den Druckverlust erhöht, oder die Heizflächen vergrößern.

In der deutschen Offenlegungsschrift 25 53 506.2 wird eine Anlage zur Erzeugung von Methan oder Synthesegas aus kohlenstoffhaltigen Stoffen mit Hilfe eines Kernreaktors beschrieben, wobei ein Teil des kohlenstoffhaltigen Stoffes unter Zugabe von Wasserdampf und bei hoher Temperatur in einem Wasserdampfvergaser zu Synthesegas umgewandelt wird. Diese Anlage hat zwei Reaktorkühlkreise, wobei in einem ersten Kühlkreis der Wasserdampfvergaser angeordnet ist und in einem zweiten Kühlkreis ein an sich bekannter Spaltofen angeordnet ist, in dem ein Teil des erzeugten Methans bei hoher Temperatur zu Wasserstoff gespalten wird, der zur hydrierenden Vergasung eines anderen Teils des kohlenstoffhaltigen Stoffes benutzt wird. Diese zweistufige Vergasung verbindet in vorteilhafter Weise die Anforderungen: niedriger Kohleverbrauch, vollständige Vergasung der Kohle, Einbindung nahezu der gesamten Kernreaktorwärme in den Vergasungsprozeß, so daß fast kein Strom nach außen abgegeben werden muß, und hoher Gesamtwirkungsgrad. Nachteilig ist dabei noch, daß für den Wasserdampfvergaser die notwendige Beheizung bei hoher Temperatur entweder direkt oder über einen Zwischenkreislauf vom nuklear beheizten Helium bereitgestellt werden muß. Dieses Helium müßte eine Temperatur von ca. 900—950°C haben und die Anlagenteile zwischen Kernreaktor und Vergasungsanlage müssen für diese hohe Temperatur geeignet sein. Der Helium-Sekundärkreislauf, der die Wärme des Kernreaktors von dessen Primärkreislauf auf die Vergasungsanlage überrägt, ist mit einem erheblichen Aufwand und auch mit zusätzlichen Verlusten verbunden. Die enge Kopplung zwischen Kernreaktor- und Vergasungsanlage bedingt einen erheblichen sicherheitstechnischen Aufwand und erschwert den Betrieb des Gesamtanlage.

In der deutschen Offenlegungsschrift 27 04 465 wird eine Anlage zur hydrierenden Vergasung von Kohle beschrieben, in der ein Teil des gewonnenen Methans vermittels eines Spaltofens in Wasserstoff und Kohlenmonoxyd zerlegt wird. Aus wärmewirtschaftlichen Gründen wird dort vorgeschlagen, den bei der hydrierenden Vergasung abfallenden Restkoks zur Beheizung des Spaltofens zu nutzen, indem dieser Restkoks entweder direkt verbrannt oder auf dem Umwege über einen sogenannten Schwachgasvergaser verwertet wird.

Aufgabe der vorliegenden Erfindung ist eine Anlage zur Erzeugung von Methan oder Synthesegas aus kohlenstoffhaltigen Stoffen ohne deren Verbrennung, die die beschriebenen Nachteile weitgehend vermeidet und von einer Energieversorgungsanlage weitgehend entkoppelt ist, so daß Störungen an der einen Anlage sich nicht unmittelbar auf die andere Anlage auswirken können. Außerdem soll diese Anlage keine wesentlichen Anteile an elektrischem Strom von außen beziehen oder nach außen abgeben. Weiterhin sollen bei der Synthesegaserzeugung durch elektrische Beheizung die Prozeßtemperatur im Methanspaltofen auf ca. 1000°C angehoben werden, was. z.Z. mit rein nuklearer Beheizung nicht realisiert werden kann. Bei dieser Temperatur laufen die chemischen Prozesse vollständig ab, so daß das Produktgas nur noch geringe Anteile an nicht umgesetztem Methan enthält und der Aufwand für Reinigungs und Zerlegungsapparate geringer wird. In der Endstufe des Wasserdampfvergasers soll die elektrisch erzeugte hohe Temperatur einen weitgehenden Umsatz des Kohlenstoffs bewirken.

Zur Lösung dieser Aufgabe wird eine Anlage nach dem ersten Anspruch vorgeschlagen. Die gegenseitige Beheizung der beiden Vergaser erspart einen in der bekannten Anlage dargestellten zweiten Heizkreislauf und zwar aus folgendem Grund:

Durch die exotherme Reaktion der hydrierenden Vergasung ist est möglich, ohne Wärmezufuhr von außen diese Vergasungsstufe auf höherem Temperaturniveau als die folgende Wasserdampfvergasung ablaufen zu lassen und damit das Rohgas von der hydrierenden Vergasung zur indirekten Beheizung der endothermen Wasserdampfvergasung zu benutzen. Bei der bekannten Anlage muß an dieser Stelle Hochtemperaturwärme eines Kernreaktors eingebracht werden. Da die Rohgase aus dem hydrierenden und dem Wasserdampfvergaser jeweils mit einer sehr hohen Temperatur austreten und es unwirtschaftlich ist, diese hohe Temperatur wie bisher nur zur Vorwärmung des eintretenden Rohgases oder nur zur Dampferzeugung auszunutzen, ist mit dieser Schaltung ein guter Gesamtwirkungsgrad der Anlage zu erwarten. Die Anforderungen an die Dichtigkeit eines Wärmetauschers, insbesondere im Bereich des Wasserdampfvergaser, sind erheblich geringer, wenn auf beiden Seiten nur Prozeßgase geführt werden und nicht ein Reaktorkühlmittel. Der wesentlichste Vorteil dieser Anlage ist aber, daß die Vergasungsanlage weitgehend von ihrer Energieversorgung, insbesonderer einer Kernreaktoranlage entkoppelt ist. Kernenergieanlagen werden aus Sicherheitsgründen in einem sogenannten Sicherheitsbehälter angeordnet, der alle radioaktiven Kreisläuge umschließt, und der bei einem Schandensfall vollständig verschlossen werden kann. Dieser Sicherheitsbehälter kann von Leitungen für Wasser bsw. Dampf durchdrungen werden. Die kontinuierliche Beschickung mit großen Mengen von Feststoffen und die Ausschleusung des Restkokses oder der Asche ist aber mit einem erheblichen Aufwand verbunden. Außerdem kann der Vergaser unabhängig vom Reaktor mit seinem günstigsten Betriebsdruck also z.B. 80 bar betrieben werden.

Diese Anlage hat als einzige Kopplung zwischen Vergasungsanlage und Energieversorgungsanlage einen Dampferzeuger, der bei einem Kernreaktor mit geringem Aufwand innerhalb des Sicherheitsbehälters angeordnet werden kann und auch ohne einen Sekundärkühlkreis unmittelbar vom Primärmedium beheizt werden kann. Der bei diesen Anlage übliche Methanspaltofen kann weggelassen werden, wenn man nur Methan produzieren will. Wenn man auch den Wasserdampfvergaser teilweise elektrisch beheizt, kann man den Kernreaktor, der sowohl ein Hochtemperaturreaktor als auch ein Druckwasserreaktor sein kann, räumlich von der Vergaseranlage trennen. Als einzige Leitungen, die den Sicherheitsbehälter des Kernreaktors durchdringen müssen, sind nur noch Wasser- bzw. Dampfleitungen vorhanden, deren Technologie bei Kernreaktoranlagen inzwischen zum gesicherten Stand der Technik gehört. Die Energieübertragung vom Kernreaktor über Dampferzeuger, Turbine und Generator bis zur elektrischen Beheizung ist naturgemäß gegenüber der direkten Beheizung mit einem schlechteren Wirkungsgrad verbunden. Da aber nur ein Teil der Kernenergie zur Stromerzeugung verwendet wird und ein großer Teil der Kernenergie ohne wesentlichen Verluste als Prozeßdampf in die Vergasungsanlage eingeführt wird, hat die Gesamtanlage einen guten Wirkungsgrad bei einem wesentlich geringeren Investitionsaufwand. Unter diesen Umständen kann man es sich sogar leisten, die Kernenergie beispielsweise mit einem Druckwasserreaktor bereitzustellen. Die vorgeschlagene elektrische Beheizung ist besonders geeignet zur Vergasung des Restkoks, der ja schon große Ascheanteile enthält und dementsprechend träge reagiert.

Die Anlage nach dem 2. Anspruch dient zur Produktion von Synthesegas und hat daher einen Methanspaltofen, der aber anders als bisher elektrisch beheizt wird. Hier zeigt sich ein besonderer betriebstechnischer Vorteil der elektrischen Beheizung gegenüber der direkten Beheizung mit Helium. Der notwendige Katalysatorwechsel kann durchgeführt werden, ohne den Reaktor abzuschalten, weil bei Anlagen dieser Art sicher zahlreiche parallel geschalteten Spaltöfen vorhanden sind, von denen jeweils einer von der Anlage getrennt und gewartet werden kann, ohne die übrige Anlage zu stören. Zudem können Kernreaktor, Vergaser und Spaltofen unabhängig voneinander jeweils mit dem günstigsten Druck betrieben werden.

Die Figur 1 zeigt ein stark schematisiertes Beispiel der Erfindung. Die gemahlene und getrocknete Kohle wird bei 1 dem hydrierenden Vergaser 2 zugeführt und dort hydrierend vergast. Der dort abfallende Restkoks wird auf dem Weg 3 dem in seiner Endstufe elektrisch beheizten Wasserdampfvergaser 4 zugeführt und weiter vergast. Der dort abfallende Rückstand besteht aus Asche mit einem geringen Anteil Kohlenstoff und wird bei 5 abgezogen. Das aus dem hydrierenden Vergaser 2 austretende Rohgas gibt einen Teil seiner Wärme im oberen Temperaturbereich im Wasserdampfvergaser 4 ab und im unteren Temperaturbereich im Prozeßdampfüberhitzer 6 und im Danpferzeuger 7. Nach Abkühlung wird das Rohgas bei 8 von Staub und Teer sowie in einer Gaswäsche von Kohlendioxyd und

3

Schwefelwasserstoff befreit. In der Tieftemperaturzerlegung 9 wird das gereinigte Gas zerlegt in eine Methan-Fraktion 10, in eine Wasserstoff-Fraktion 11 und eine Kohlenmonoxyd-Fraktion 12. Das Methan wird einem Gasnetz zugeführt. Das den Wasserdampfvergaser 4 auf dem Wege 14 verlassende Hydriergas gibt einen Teil seiner Wärme an das dem hydrierenden Vergaser 2 zuzuführende Hydriergas 15 in dem Wärmetauscher 16 ab. Das im Hydriergas enthaltene Kohlenmonoxyd wird in der Konvertierung 17 mit Wasserdampf in Wasserstoff und Kohlendioxyd überführt. Danach wird das Hydriergas in dem Abhitzekessel 18 weiter abgekühlt, wobei Prozeßdampf erzeugt wird. Nachdem in der Gaswäsche 19 Kohlendioxyd und Schwefelwasserstoff entfernt wurden, wird das derart gereinigte Gas mit dem aus der Tieftemperaturzerlegung 9 abgegebenen Wasserstoff auf dem Wege 15 im Wärmetauscher 16 vorgewärmt und dem hydrierenden Vergaser 2 zugeleitet, wo es einen großen Teil des Kohlenstoffs in Methan verwandelt. Die gesamte Wärmeenergie wird von einem Kernreaktor 23 bereitgestellt, dessen geschlossener Primärkreislauf mit einem Gebläse oder einer Pumpe 24 betrieben wird und der seine verwertbare Wärme an einen Dampferzeuger 25 abgibt.

Figur 2 zeigt ein weiteres stark schematisiertes Beispiel der Erfindung anhand einer Kohlevergasungsanlage, die im wesentlichen zur Herstellung von Synthesegas dient und daher einen Methanspaltofen aufweist. Gegenüber der in Figur 1 bereits beschriebenen Anlage ist hier noch ein Methanspaltofen 27 vorhanden, der in seinem oberen Temperaturbereich mit elektrischem Strom aus der Dampfturbinenanlage 21 beheizt wird, und der bei 20 mit einem Gemisch aus Methan und Wasserdampf beschickt wird. Das austretende gespaltene Rohgas dient im Kreuzgegenstrom zur Beheizung des eintretenden Methan- Wasserdampfgemisches und wird dem aus dem Wasserdampfvergaser austretenden Rohgas zwischen dem Prozeßdampferhitzer 6 und dem Prozeßdampferzeuger 7 zugeführt. Ebenfalls im Unterschied zu Figur 1 wird in Figur 2 nicht Methan nach außen abgegeben, sondern Synthesegas als Gemisch aus dem Wasserstoffstrom 11 und dem Kohlenmonoxydstrom 12.

Die folgenden Zahlenbeispiele beziehen sich auf eine erfindungsgemäße Kohlevergasungsanlage, die mit einem Kernreaktor beheizt wird.

**0 016 179**

Beispiele

| | |
|---|---|
| Thermische Reaktorleistung: | 1 500 MW |
| Kohleart: Gasflammkohle mit 38% flüchtigen Bestandteilen | |
| Brennwert des erzeugten Methans: | 40 693 KJ/$m^3_N$ |
| Zusammensetzung: | 95% $CH_4$ und 5% $C_2H_6$ |

Maximal 60% der Kohle werden hydrierend vergast.

### Beispiel 1
Hochtemperaturreaktor mit elektrisch beheiztem Spaltofen

| | |
|---|---|
| Kohledurchsatz: | 525 t/h |
| Methanerzeugung: | 379 · $10^3$ $m^3_N$/h |
| Stromverbrauch: | |
| Vergaser | 147 MW |
| Spaltofen | 74 MW |
| Gasfabrik | 259 MW |
| Thermische Reaktorleistung für Prozeßdampf | 115 MW |

### Beispiel 2
Leichtwasserreaktor mit elektrisch beheizten Spaltofen

| | |
|---|---|
| Kohledurchsatz: | 442 t/h |
| Methanerzeugung: | 319 · $10^3$ $m^3_N$/h |
| Stromverbrauch: | |
| Vergaser | 124 MW |
| Spaltofen | 62 MW |
| Gasfabrik | 216 MW |
| Thermische Reaktorleistung für Prozeßdampf | 283 MW |

### Beispiel 3
Leichtwasserreaktor ohne Methanspaltofen

| | |
|---|---|
| Kohledurchsatz: | 452 t/h |
| Methanerzeugung: | 327 · $10^3$ $m^3_N$/h |
| Stromverbrauch: | |
| Vergaser | 164 MW |
| Gasfabrik | 221 MW |
| Thermische Reaktorleistung für Prozeßdampf | 333 MW |

In allen Beispielen wird die Endstufe des Wasserdampfvergasers elektrisch beheizt und kein Strom nach außen abgegeben.

## 0016179

**Patentansprüche**

1. Anlage zur Erzeugung von Methan oder Synthesegas aus kohlenstoffhaltigen Stoffen ohne Verbrennung dieser Stoffe mit einem hydrierenden Vergaser (2), in dem ein erster Teil des Kohlenstoffs mit Wasserstoff zu Methan umgesetzt wird und einem Wasserdampfvergaser (4) in dem ein zweiter Teil des Kohlenstoffs mit Wasserdampf zu Synthesegas umgesetzt wird, gekennzeichnet durch folgende Merkmale:

a) Wärmetauschkanäle im Wasserdampvergaser (4), in denen das aus dem hydrierenden Vergaser (2) austretende Rohgas Wärme abgibt.

b) Einem Wärmetauscher (16), in dem das aus dem Wasserdampfvergaser (4) strömende Rohgas Wärme an das in den hydrierenden Vergaser (2) eintretende Wasserstoffgas abgibt.

c) Die Endstufe des Wasserdampfvergasers (4) hat eine elektrische Beheizung.

2. Anlage zur Herstellung von Synthesegas nach Anspruch 1 mit einem Methanspaltofen (27), gekennzeichnet durch folgendes Merkmal:

a) Der Methanspaltofen (27) hat im oberen Temperaturbereich eine elektrische Beheizung.

**Revendications**

1. Installation de production de méthane ou de gaz de synthèse à partir de matières contenant du carbone sans combustion de ces matières, comprenant un gazéificateur par hydrogénation (2), dans lequel une première partie du carbone est transformée en méthane par l'hydrogène et un gazéificateur à la vapeur d'eau (4), dans lequel une seconde partie du carbone est transformée en gaz de synthèse par de la vapeur d'eau, caractérisée par les caractéristiques suivantes:

a) des canaux d'échange de chaleur dans le gazéificateur à la vapeur d'eau (4) dans lesquels le gaz brut sortant du gazéificateur par hydrogénation (2) cède de la chaleur.

b) Un échangeur de chaleur (16), dans lequel le gaz brut sortant du gazéificateur à la vapeur d'eau (4) cède de la chaleur à l'hydrogène gazeux entrant dans le gazéificateur par hydrogénation.

c) L'étage final du gazéificateur à la vapeur d'eau a un chauffage électrique.

2. Installation de préparation de gaz de synthèse suivant la revendication 1 comprenant un four de dissociation de méthane (27), caractérisée par la caractéristique suivante:

a) le four de dissociation du méthane (27) a un chauffage électrique dans la plage des températures élevées.

**Claims**

1. Apparatus for producing methane or synthesis gas from carbonaceous substances without combustion of the latter, comprising a hydrogenating gasifier (2), in which a first part of the carbon is converted into methane with hydrogen, and a steam gasifier (4), in which a second part of the carbon is converted into synthesis gas with steam, characterised by the following features:

a) heat exchange channels in the steam gasifier (4) in which the crude gas issuing from the hydrogenating gasifier (2) gives up heat;

b) a heat exchanger (16) in which the crude gas flowing from the steam gasifier (4) gives up heat to the incoming hydrogen gas to the hydrogenating gasifier (2);

c) the final stage of the steam gasifier (4) is electrically heated.

2. Apparatus for the production of synthesis gas according to Claim 1, having a methane cracking furnace (27), characterised by the following feature:

a) the methane cracking furnace (27) is electrically heated in the upper temperature range.

FIG 1

H₂  11

12  CO  10  CH₄  9

CO₂S H₂S  19  8  CO₂S H₂S

18  7

17  6

15  16  14

1  2  3  5

21  25

23  24

FIG 2